# EUROPEAN PATENT APPLICATION

(11) **EP 4 659 743 A1**
(43) Date of publication of application: **10.12.2025**
(21) Application number: 24750159.6
(22) Date of filing: 26.01.2024
(51) Int. Cl.: A61K 9/20, A61K 9/16, A61K 47/10, A61K 47/14, A61K 47/36, A61K 47/44

(54) **SELF-EMULSIFYING COMPOSITION AND PRODUCTION METHOD THEREOF**

(30) Priority: 30.01.2023 JP 2023011592
(71) Applicant: Sunsho Pharmaceutical Co., Ltd., Fuji-shi, Shizuoka 419-0201 (JP)
(72) Inventor: OZEKI Toshinori, Fuji-shi, Shizuoka 419-0201 (JP); MINEDA Misuzu, Fuji-shi, Shizuoka 419-0201 (JP); OOKAWARA Masaki, Fuji-shi, Shizuoka 419-0201 (JP); SEI Shunsuke, Fuji-shi, Shizuoka 419-0201 (JP); KURONO Masahiro, Fuji-shi, Shizuoka 419-0201 (JP); SUGIURA Mizue, Fuji-shi, Shizuoka 419-0201 (JP)
(74) Representative: TBK
(86) International application number: PCT/JP2024/002428
(87) International publication number: WO 2024/162210

(57) **Abstract**

Provided is a self-emulsifying formulation or the like in a novel form, capable of forming a dosage form such as tablet. The self-emulsifying composition comprises a core particle comprising a useful substance, an oily ingredient, a surfactant, and a thickener; and a coating particle layer which coats the core particle.

## Description

### Technical Field

The present invention relates to a self-emulsifying composition and a method of producing the same.

### Background Art

Among the routes of administration for pharmaceuticals and the like, oral administration is the most widely used because it allows for safe and convenient delivery of pharmaceuticals and the like. However, in such cases where useful substances are poorly water-soluble or unstable, there tends to be variable in their absorption behavior when administered orally, leading to inter-individual variability.

Therefore, conventional technologies for orally administering poorly water-soluble useful substances, unstable useful substances and the like by using Self-Emulsifying Drug Delivery Systems (SEDDS), known as self-emulsifying formulations, have been explored. Self-emulsifying formulations generally refer to preparations which spontaneously form emulsions upon contact with moisture such as saliva and digestive fluids during oral administration.

For example, Patent Literature 1 describes an invention related to metronomic oral gemcitabine for cancer therapy, which involves using a formulation containing gemcitabine (GEM) dissolved in a hydrophilic solvent, a predetermined surfactant, and a predetermined hydrophilic carrier. According to the invention described in Patent Literature 1, it is described that gemcitabine (GEM) can be administered orally in its original form in a metronomic manner without being converted into a prodrug. In addition, Patent Literature 1 describes that when the formulation is orally administered, the formulation naturally forms an emulsion upon contact with an aqueous medium at 37°C under mild mechanical agitation, similar with that in the human gastrointestinal tract.

### Citation List

### Patent Literature

Patent Literature 1
Japanese Translation of PCT International Application Publication No. 2019-517490

### Summary of Invention

### Technical Problem

Self-emulsifying formulations, by their nature, need to be in form of liquid or soft capsules. However, these dosage forms require special techniques during production and may also necessitate careful handling.

Therefore, there is a need for a novel type of self-emulsifying formulation which can be made into dosage forms such as tablet.

### Solution to Problem

The present invention can have the following aspects for example.

[1] A self-emulsifying composition comprising:
   a core particle comprising an oily ingredient, a surfactant and a thickener; and
   a coating particle layer which coats the core particle.
[2] The self-emulsifying composition according to [1], wherein the oily ingredient contains at least one selected from the group consisting of hydrogenated vegetable oils, medium-chain fatty acid triglycerides (MCT), long-chain fatty acid alkyl esters, and propylene glycol mono medium-chain fatty acid esters.
[3] The self-emulsifying composition according to [1] or [2], wherein the surfactant contains at least one selected from the group consisting of polyoxyethylene fatty acid glyceryl esters, polyoxyethylene hydrogenated castor oils, and polyoxyethylene castor oils.
[4] The self-emulsifying composition according to any one of [1] to [3], wherein the thickener contains at least one selected from the group consisting of natural thickeners, high molecular weight PEG, glycerin fatty acid esters, and polyoxyethylene polyoxypropylene glycols (POE-POPG).
[5] The self-emulsifying composition according to [1] to [4], further comprising a useful substance.
[6] The self-emulsifying composition according to [5], wherein the useful substance has a molecular weight of 500 or more.
[7] The self-emulsifying composition according to [5] or [6], wherein the useful substance has a logarithmic value of octanol/water partition coefficient (Log P value) of 3 or higher.
[8] The self-emulsifying composition according to any one of [1] or [7], further comprising a solvent.
[9] The self-emulsifying composition according to [8], wherein the solvent contains at least one selected from the group consisting of low molecular weight PEG and polyalcohols.
[10] A solid preparation comprising the self-emulsifying composition according to any one of [1] to [9].
[11] The solid preparation according to [10], which is tablet or granule.
[12-1] A method of producing a self-emulsifying composition, comprising a granulation step of introducing droplets of a granulation solution comprising an oily ingredient, a surfactant, and a thickener into a fluidized bed where coating particles flow to attach the coating particles onto the surface of the droplets for granulation.
[12-2] A method of producing the self-emulsifying composition according to any one of [1] to [11], comprising a granulation step of introducing droplets of a granulation solution containing an oily ingredient, a surfactant, and a thickener into a fluidized bed where coating particles flow to attach the coating particles onto the surface of the droplets for granulation.

### Advantageous Effects of Invention

The present invention provides a novel type of self-emulsifying formulation and the like.

### Brief Description of Drawings

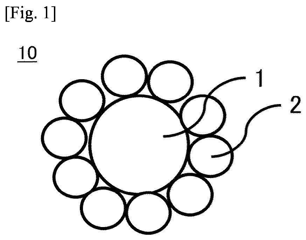

[Figure 1] Figure 1 is a schematic diagram of a self-emulsifying composition according to an embodiment of the present invention.

### Description of Embodiments

Hereinafter, embodiments for carrying out the present invention are described in detail.

### 1 Self-emulsifying composition

The self-emulsifying composition according to the present invention comprises: a core particle comprising an oily ingredient, a surfactant and a thickener; and a coating particle layer which coats the core particle. Furthermore, as used herein, "self-emulsifying composition" refers to that which spontaneously can form emulsions upon contact with moisture such as saliva and digestive fluids during oral administration, and comprises, not only Self-Emulsifying Drug Delivery System (SEDDS), but also Self-Microemulsifying Drug Delivery System (SMEDDS), Self-Nanoemulsifying Drug Delivery System (SNEDDS) and the like.

The self-emulsifying composition according to the present invention can be formulated into dosage forms such as tablet or granule.

Hereinafter, the present invention is explained with reference to the drawings. Furthermore, the drawings may be exaggerated for the sake of explanation and may differ from actual dimensions.

Figure 1 is a schematic diagram of a self-emulsifying composition according to one embodiment of the present invention.

The self-emulsifying composition 10 has; the core particle 1 which comprises an oily ingredient, a surfactant, a thickener and a useful substance; and the coating particle layer 2 which coats the core particle 1. By having the coating particle layer 2 formed by attaching the coating particles onto the surface of the core particle 1, it is possible to granulate the self-emulsifying composition 10 into a defined form.

In one embodiment of the present invention, when a solid preparation (for example, a tablet) containing self-emulsifying composition 10 is taken, the self-emulsifying composition 10 contacts with moisture present in the digestive tract, such as saliva in the oral cavity and gastric fluid in the stomach, and naturally forms water-in-oil (o/w) emulsions.

Here, the emulsion droplets originate from the ingredients of the core particles, and the emulsion droplets contain oily ingredients, surfactants, thickeners, useful substances, and the like. Also, the emulsion droplets are evenly dispersed within the digestive tract. As a result, even for poorly water-soluble useful substances, unstable useful substances, or the like, the absorption behavior stabilizes and the occurrence of inter-individual variability can be suppressed.

Also, when useful substances are poorly water-soluble, the ingredients of the self-emulsifying composition (oily ingredients, surfactants, thickeners, etc.) can enhance the solubility of the useful substances in water (moisture such as saliva and digestive fluids, and the like). As a result, water-insoluble useful substances become easier to be absorbed. Furthermore, as used herein, "poorly water-soluble" means that the logarithm of the octanol/water partition coefficient (Log P value) is 3 or higher.

Furthermore, the mean particle diameter of the emulsion droplets is preferably 0.1 nm to 10 µm; more preferably 3 to 500 nm; even more preferably 5 to 300 nm; and particularly preferably 20 to 200 nm. Also, the polydispersity index of the emulsion droplets is preferably less than 0.7; more preferably 0.1 to 0.6; even more preferably 0.1 to 0.5; and particularly preferably 0.1 to 0.3. Furthermore, as used herein, the "mean particle diameter" and "polydispersity index" of the emulsion droplets are measured using the method described in the examples.

Also, the self-emulsifying composition 10 according to one embodiment of the present invention is in a solid form having the core particle 1 and the coating particle layer 2, and can thus be suitably formulated into dosage forms such as tablet and granule.

The mean particle diameter of the self-emulsifying composition is preferably 10 to 1000 µm; and more preferably 100 to 500 µm. When the mean particle diameter of the self-emulsifying composition is 10 µm or more, sufficient hardness can be obtained, allowing the form to be maintained during formulation processes such as tableting, which is preferable. On the other hand, when the mean particle diameter of the self-emulsifying composition is less than 1000 µm, the resulting tablets and the like can be of a size for easy intake, which is preferable. Furthermore, as used herein, the "particle diameter" refers to the maximum distance between two points on the outline of the object, unless stated otherwise. Also, the "mean particle diameter" refers to the average particle diameter of any 50 objects contained within a single field of view of a scanning electron microscope (SEM), unless stated otherwise.

Hereinafter, each component of the composition of the present invention is described in detail.

### [Core particle]

The core particle comprises an oily ingredient, a surfactant and a thickener. It can further comprise other useful substance, solvent, water, additive and the like.

The mean particle diameter of the core particle is preferably 5 to 1000 µm; and more preferably 100 to 500 µm. When the mean particle diameter of the core particle is 5 µm or more, it allows for a higher proportion of core particles, which is preferable. On the other hand, when the mean particle diameter of the core particle is 1000 µm or less, the resulting tablets and the like can be of a size for easy intake, which is preferable.

### (Oily ingredient)

Oily ingredients have functions such as serving as medium for the ingredients contained within the core particles; functioning as dispersoid (droplets) of emulsion when an oral dosage of self-emulsifying composition or the like is administered; and enhancing the solubility of useful substances in water.

Examples of oily ingredients include, but are not limited to, vegetable oils, animal fats, and derivatives thereof, fatty acid glycerides, fatty acid alkyl esters, polyglycerin fatty acid esters, propylene glycol fatty acid esters.

Examples of vegetable oils include, but are not limited to, soybean oil, rapeseed oil, seed oil, sunflower oil, peanut oil, palm oil, palm kernel oil, coconut oil, sesame oil, castor oil, olive oil, corn oil, linseed oil, macadamia nut oil, meadowfoam oil, safflower oil, avocado oil, canola oil, apricot kernel oil, rice germ oil, rice bran oil.

Examples of animal fats include, but are not limited to, beef tallow, lard, fish liver oil, fish oil, whale oil.

Examples of derivatives of vegetable oils or animal fats include, but are not limited to, hydrogenated vegetable oils or animal fats (such as hydrogenated soybean oil and hydrogenated castor oil).

Examples of fatty acid triglycerides include medium-chain fatty acid triglycerides (MCT) containing medium-chain fatty acids with carbon number of 8 to 12 (such as caprylic acid, capric acid, and lauric acid) such as glycerol caprylate, glycerol caprate, glycerol laurate, glycerol tri(caprylate-caprate), glycerol tri(caprylate-laurate), glycerol tri(caprate-laurate), glycerol tri(caprylate-caprate-laurate); and long-chain fatty acid triglycerides containing long-chain fatty acids with carbon number of 13 or more (such as myristic acid, palmitic acid, stearic acid, oleic acid, linolenic acid, linoleic acid, alpha-linolenic acid, behenic acid, erucic acid) such as glycerol trimellitate, glycerol tristearate, glycerol trioleate, and glycerol trilinoleate.

Examples of fatty acid alkyl esters include medium-chain fatty acid alkyl esters with carbon number of 8 to 12 such as ethyl caprylate, ethyl caprate, and ethyl laurate; and long-chain fatty acid alkyl esters such as ethyl stearate, and ethyl oleate.

Examples of polyglycerin fatty acid esters include polyglycerin medium-chain fatty acid esters such as polyglyceryl caprylate, polyglyceryl caprate, and polyglyceryl laurate; and polyglycerin long-chain fatty acid esters such as polyglyceryl stearate and polyglyceryl oleate.

Examples of propylene glycol fatty acid esters include propylene glycol mono medium-chain fatty acid esters such as propylene glycol monocaprylate and propylene glycol monolaurate; and propylene glycol mono long-chain fatty acid esters such as propylene glycol palmitate and propylene glycol stearate.

Among these, the oily ingredients preferably contain at least one selected from the group consisting of derivatives of plant oils, fatty acid triglycerides, fatty acid alkyl esters, and propylene glycol fatty acid esters; more preferably contain at least one selected from the group consisting of hydrogenated vegetable oils, medium-chain fatty acid triglycerides (MCT), long-chain fatty acid alkyl esters, and propylene glycol mono medium-chain fatty acid esters; and even more preferably contain medium-chain fatty acid triglycerides (MCT).

In one embodiment, the oily ingredient preferably contains at least one selected from the group consisting of fatty acid triglycerides, fatty acid alkyl esters, and propylene glycol fatty acid esters; more preferably contains at least one selected from the group consisting of medium-chain fatty acid triglycerides (MCT), long-chain fatty acid alkyl esters, and propylene glycol mono medium-chain fatty acid esters; from the perspective of making the mean particle diameter of emulsion droplets smaller, even more preferably contains at least one selected from the group consisting of glyceryl trilaurate, ethyl oleate, propylene glycol monocaprylate, and propylene glycol monolaurate; and particularly preferably contains at least one selected from the group consisting of ethyl oleate and propylene glycol monocaprate.

In another embodiment, the oily ingredient preferably contains at least one selected from the group consisting of fatty acid triglycerides, polyglycerin fatty acid esters, and propylene glycol fatty acid esters; from the perspective of enhancing the solubility of useful substances which are poorly soluble in water to enhance their absorbability, more preferably contains at least one selected from the group consisting of fatty acid triglycerides and propylene glycol fatty acid esters; even more preferably contains propylene glycol fatty acid esters; and particularly preferably contains propylene glycol monocaprylate (mono-caprylic acid propylene glycol).

These oily ingredients can be used alone or in combination with two or more types.

The content of the oily ingredient, relative to the total mass of the self-emulsifying composition, is preferably 0.1 to 30% by mass; more preferably 0.5 to 20% by mass; and even more preferably 1 to 15% by mass. Furthermore, when two or more types of oily ingredients are contained, it is preferable for their total content to fall within the above ranges.

Also, the content of the oily ingredient, relative to the total mass of the core particles, is preferably 0.5 to 60% by mass; more preferably 1 to 50% by mass; and even more preferably 2 to 40% by mass. Furthermore, when two or more types of oily ingredients are contained, it is preferable for their total content to fall within the above ranges.

### (Surfactant)

Surfactants have functions such as enhancing the uniformity of ingredients contained within core particles; promoting the formation of emulsions when self-emulsifying composition and the like are orally administered; and enhancing the solubility of useful substances in water.

Examples of surfactants include anionic surfactants, cationic surfactants, amphoteric surfactants, and nonionic surfactants.

Examples of anionic surfactants include, but are not limited to, potassium laurate, potassium myristate, potassium palmitate, sodium oleate, triethanolamine lauryl sulfate, sodium polyoxyethylene lauryl ether sulfate, triethanolamine polyoxyethylene lauryl ether sulfate, and sodium methyl taurine fatty acid from coconut oil.

Examples of cationic surfactants include, but are not limited to, benzalkonium chloride, cetyltrimethylammonium chloride, steartrimethylammonium chloride, and alkyltrimethylammonium chloride.

Examples of amphoteric surfactants include, but are not limited to, 2-alkyl-N-carboxymethyl-N-hydroxyethylimidazolinium betaine, lauryl dimethyl amino acetate betaine, and palm oil alkyl betaine.

Examples of nonionic surfactants include, but are not limited to, sorbitan fatty acid esters such as sorbitan monolaurate, sorbitan monopalmitate, sorbitan monostearate, and sorbitan monooleate; polyoxyethylene monoalkyl ethers such as polyoxyethylene lauryl ether, polyoxyethylene oleyl ether, polyoxyethylene cetyl ether, and polyoxyethylene stearyl ether; polyoxyethylene alkyl ether esters such as polyoxyethylene myristyl ether myristate, polyoxyethylene cetyl ether stearate, and polyoxyethylene stearyl ether stearate; polyoxyethylene fatty acid glyceryl esters such as polyoxyethylene caprylate glyceride, polyoxyethylene caprate glyceride (PEG-6 capric glyceride, etc.), polyoxyethylene (caprylate/caprate) glyceride (PEG-8 (caprylic/capric) glyceride, etc.), polyoxyethylene glyceryl laurate (PEG-12 glyceryl laurate, PEG-20 glyceryl laurate, etc.), and polyoxyethylene glyceryl oleate (PEG-20 glyceryl oleate, PEG-30 glyceryl oleate, etc.); polyoxyethylene glyceryl isostearates (PEG-8 glyceryl isostearate, PEG-10 glyceryl isostearate, PEG-15 glyceryl isostearate, PEG-20 glyceryl isostearate, etc.); polyoxyethylene glyceryl triisostearates (PEG-10 glyceryl triisostearate, PEG-20 glyceryl triisostearate, etc.); polyoxyethylene trimethylolpropane distearates (PEG-4 trimethylolpropane distearate); polyoxyethylene sorbitan mono fatty acid esters such as polysorbate 20, polysorbate 60, and polysorbate 80; polyoxyethylene sorbitan fatty acid esters such as polysorbate 65 and polysorbate 85; triethyl citrate; monoglyceride citrate; triacetin; polyoxyethylene hydrogenated castor oils (polyoxyethylene hydrogenated castor oil 20, polyoxyethylene hydrogenated castor oil 40, polyoxyethylene hydrogenated castor oil 60, etc.); polyoxyethylene hydrogenated castor oil mono fatty acid such as polyoxyethylene hydrogenated castor oil laurate; polyoxyethylene hydrogenated castor oil succinate such as polyoxyethylene hydrogenated castor oil succinate; and polyoxyethylene castor oils.

Among these, the surfactants preferably contain a nonionic surfactant; and more preferably contain at least one selected from the group consisting of polyoxyethylene fatty acid glyceryl esters, polyoxyethylene hydrogenated castor oils, and polyoxyethylene castor oils.

In one embodiment, the surfactant preferably contains a polyoxyethylene fatty acid glyceryl ester; more preferably contains at least one selected from the group consisting of polyoxyethylene caprylate glyceride, polyoxyethylene caprate glyceride, polyoxyethylene glyceryl laurate and polyoxyethylene glyceryl oleate; and even more preferably contains polyoxyethylene glyceryl oleate.

In one embodiment, the surfactant preferably contains polyoxyethylene castor oil.

In one embodiment, the surfactant preferably contains polyoxyethylene hydrogenated castor oil.

In one embodiment, the surfactant preferably contains a polyoxyethylene castor oil and a polyoxyethylene fatty acid glyceryl ester; more preferably contains polyoxyethylene castor oil and at least one selected from the group consisting of polyoxyethylene caprylate glyceride, polyoxyethylene caprate glyceride, polyoxyethylene glyceryl laurate, and polyoxyethylene glyceryl oleate; even more preferably contains a polyoxyethylene castor oil and at least one selected from the group consisting of polyoxyethylene glyceryl caprate and polyoxyethylene glyceryl oleate; and particularly preferably contains a polyoxyethylene castor oil and polyoxyethylene glyceryl caprate.

In one embodiment, the surfactant preferably contains polyoxyethylene castor oil and polyoxyethylene hydrogenated castor oil.

The surfactants described above can be used alone or in combination with two or more types.

The content of the surfactant, relative to the total mass of the self-emulsifying composition, is preferably 0.3 to 20% by mass; more preferably 0.5 to 15% by mass; and even more preferably 1 to 10% by mass. Furthermore, when two or more types of surfactants are contained, it is preferable for their total content to fall within the above ranges.

Also, the content of the surfactant, relative to the total mass of the core particles, is preferably 1 to 60% by mass; more preferably 2 to 50% by mass; and even more preferably 3 to 40% by mass. Furthermore, when two or more types of surfactants are contained, it is preferable for their total content to fall within the above ranges.

### (Thickener)

Thickeners have functions such as stabilizing the form of core particles; adjusting the physical properties (such as hardness) of self-emulsifying compositions; and enhancing the solubility of useful substances in water.

Examples of thickeners include, but are not limited to, natural thickeners such as carrageenan, gelatin, and agar; high molecular weight PEG such as polyethylene glycol 4000 (Macrogol 4000) and polyethylene glycol 6000 (Macrogol 6000); cellulose derivatives such as methyl cellulose, hydroxypropyl methylcellulose, and hydroxypropyl cellulose; copolymers such as aminoalkyl methacrylate copolymer and ammonium alkyl methacrylate copolymer; glycerin fatty acid esters such as glyceryl monostearate; polyoxyethylene monoesters such as polyethylene glycol monostearates (polyoxyl 40 stearate, etc.), polyethylene glycol monolaurates (PEG-2 laurate, PEG-4 laurate, PEG-8 laurate, etc.), polyethylene glycol monooleates (PEG-4 oleate, PEG-6 oleate, PEG-8 oleate, PEG-12 oleate, etc.), polyethylene glycol monostearates (PEG-2 stearate, PEG-9 stearate, PEG-32 stearate, etc.), and polyethylene glycol monomyristates (PEG-8 myristate, PEG-20 myristate, etc.); and polyoxyethylene polyoxypropylene glycols (POE-POPG) such as polyoxyethylene (3) polyoxypropylene (17) glycol, polyoxyethylene (20) polyoxypropylene (20) glycol, polyoxyethylene (54) polyoxypropylene (39) glycol, and polyoxyethylene (160) polyoxypropylene (30) glycol.

Among these, the thickeners preferably contain at least one selected from the group consisting of natural thickeners, high molecular weight PEG, glycerin fatty acid esters, polyoxyethylene monoesters, and polyoxyethylene polyoxypropylene glycols (POE-POPG); and more preferably contain at least one selected from the group consisting of gelatin, high molecular weight PEG, glycerin fatty acid esters, polyethylene glycol monostearates, and polyoxyethylene polyoxypropylene glycols.

In one embodiment, from the perspective of making the mean particle diameter of emulsion droplets smaller, the thickeners preferably contain polyoxyethylene monoesters; more preferably contain at least one selected from the group consisting of polyethylene glycol monostearates, polyethylene glycol monolaurates, polyethylene glycol monooleates, polyethylene glycol monostearates, and polyethylene glycol monomyristates; even more preferably contain polyethylene glycol monostearate; and particularly preferably contain polyoxyl 40 stearate.

In another embodiment, from the perspective of enhancing the solubility of useful substances which are poorly soluble in water to enhance their absorbability, the thickeners preferably contain at least one selected from the group consisting of natural thickeners, polyoxyethylene monoesters, and polyoxyethylene polyoxypropylene glycols (POE-POPG); more preferably contain at least one of natural thickeners and polyoxyethylene polyoxypropylene glycols (POE-POPG); even more preferably contain at least one of gelatin and polyoxyethylene polyoxypropylene glycols (POE-POPG); and particularly preferably contain gelatin.

The thickeners described above can be used alone or in combination with two or more types.

Furthermore, as used herein, "high molecular weight PEG" refers to polyethylene glycol having a weight average molecular weight of 2000 or more. Also "low molecular weight PEG" refers to polyethylene glycol having a weight average molecular weight of less than 2000. At the time, the average molecular weight of polyethylene glycol (PEG) is calculated based on the hydroxyl value.

The content of the thickener, relative to the total mass of the self-emulsifying composition, is preferably 5 to 70% by mass; more preferably 5 to 50% by mass; and even more preferably 10 to 50% by mass. Furthermore, when two or more types of thickeners are contained, it is preferable for their total content to fall within the above ranges.

Also, the content of the thickener, relative to the total mass of the core particles, is preferably 10 to 90% by mass; more preferably 20 to 90% by mass; and even more preferably 30 to 90% by mass. Furthermore, when two or more types of thickeners are contained, it is preferable for their total content to fall within the above ranges.

### [Useful substance]

The core particle can further comprise useful substances. In one preferable embodiment, core particles comprise useful substances.

Useful substances are preferably, but not limited to, the useful substances which are poorly water-soluble or unstable in water.

Examples of useful substances include medicinal ingredients, nutritional ingredients, food ingredients, microorganisms, and labeling substances. These useful substances include, for example, insulin, calcitonin, angiotensin, vasopressin, desmopressin, LH-RH (luteinizing hormone-releasing hormone), somatostatin, glucagon, oxytocin, gastrin, cyclosporine, tacrolimus, somatomedin, secretin, h-ANP (human atrial natriuretic peptide), ACTH (adrenocorticotropic hormone), MSH (melanocyte-stimulating hormone),β-endorphin, muramyl dipeptide, enkephalin, neurotensin, bombesin, VIP (vasoactive intestinal peptide), CCK-8 (cholecystokinin-8), PTH (parathyroid hormone), CGRP (calcitonin gene-related peptide), TRH (thyrotropin-releasing hormone), endothelin, hGH (human growth hormone), cytokines such as interleukin, interferon, colony-stimulating factor, and tumor necrosis factor, GLP-1 receptor agonists (semaglutide), cyanocobalamin, macrolide antibiotics such as azithromycin, clarithromycin, erythromycin, telithromycin, fidaxomicin, rapamycin (sirolimus), and roxithromycin, HIV protease inhibitors such as ritonavir and indinavir ethanol adduct, rifamycin antibiotics such as rifampicin, rifabutin, and rifapentine, polyene antifungal agents such as amphotericin B, nystatin, and torikomysin, cardiac glycosides such as digoxin, methyl digoxin, and lanatoside C, azole antifungal agents such as econazole, miconazole, clotrimazole, ketoconazole, itraconazole, and fluconazole, vinca alkaloids such as vincristine, vinblastine, vindesine, and vinorelbine, taxoids such as paclitaxel and docetaxel, dihydropyridine calcium channel blockers such as amlodipine, felodipine, nicardipine, nifedipine, nisoldipine, nitrendipine, lacidipine, nilvadipine, barnidipine, lercanidipine, cilnidipine, benidipine, azelnidipine, lemildipine, and teludipine, and compounds for evaluating intestinal permeability such as fluorescein isothiocyanate-dextran 4 (FITC-dextran 4: FD-4).

These useful substances can be used alone or in combination with two or more types.

In one embodiment, the useful substance has a molecular weight of preferably 500 to 1000000; more preferably 500 to 200000; and even more preferably 500 to 10000. Furthermore, as used herein, "the molecular weight of a useful substance" is measured by methods such as light scattering method, osmotic pressure method, viscosity method, and size exclusion chromatography.

Also, in another embodiment, the useful substance has a logarithmic value of octanol/water partition coefficient (Log P value) of preferably 3 or higher; more preferably 3.5 or higher; and even more preferably 4 to 8. Furthermore, as used herein, "the logarithmic value of octanol/water partition coefficient (Log P value) of a useful substance" is measured using the flask shaking method (with the partition coefficient estimated by calculation).

Although depending on the type of useful substances, the content of useful substances, relative to the total mass of the self-emulsifying composition, is preferably 0.01 to 5% by mass; more preferably 0.03 to 3% by mass; even more preferably 0.05 to 1% by mass; and particularly preferably 0.15 to 0.8% by mass.

In another embodiment, the content of useful substances, relative to the total mass of the self-emulsifying composition, is preferably 3 to 50% by mass; more preferably 5 to 40% by mass; and even more preferably 10 to 30% by mass.

Also, although depending on the type of useful substances, the content of useful substances, relative to the total mass of the core particles, is preferably 0.01 to 3% by mass; more preferably 0.02 to 2% by mass; and even more preferably 0.03 to 0.5% by mass.

In another embodiment, the content of useful substances, relative to the total mass of the core particles, is preferably 3 to 50% by mass; more preferably 5 to 40% by mass; and even more preferably 10 to 30% by mass.

Furthermore, when two or more types of useful substances are contained, it is preferable for their total content to fall within the above ranges.

### (Solvent)

The core particle can further comprise solvents. Solvents have functions such as enhancing the uniformity of ingredients contained within core particles; adjusting the hardness and the like of self-emulsifying compositions; and promoting the formation of emulsions when self-emulsifying composition and the like are orally administered.

Examples of solvents include, but are not limited to, ethanol; low molecular weight PEG such as polyethylene glycol 200 (Macrogol 200), polyethylene glycol 300 (Macrogol 300), polyethylene glycol 400 (Macrogol 400), and polyethylene glycol 600 (Macrogol 600); and polyalcohol such as propylene glycol and glycerol. Furthermore, water is not included in solvents. Furthermore, as used herein, "polyalcohol" refers to alcohols which have 2 or more of, preferably 2 to 4 of, more preferably 2 to 3 of, and even more preferably 2 of hydroxy groups in their molecules.

Among these, the solvents preferably contain at least one selected from the group consisting of low molecular weight PEG and polyalcohol; and more preferably contain at least one selected from the group consisting of low molecular weight PEG and propylene glycols.

The solvents described above can be used alone or in combination with two or more types.

The content of the solvent, relative to the total mass of the self-emulsifying composition, is preferably 0.1 to 10% by mass; more preferably 0.1 to 5% by mass; and even more preferably 0.3 to 3% by mass. Furthermore, when two or more types of solvents are contained, it is preferable for their total content to fall within the above ranges.

Also, the content of the solvent, relative to the total mass of the core particles, is preferably 0.3 to 30% by mass; more preferably 0.5 to 20% by mass; and even more preferably 1 to 10% by mass. Furthermore, when two or more types of solvents are contained, it is preferable for their total content to fall within the above ranges.

### (Water)

The core particle can further comprise water. Water has functions such as enhancing the uniformity of ingredients contained within core particles; and adjusting the hardness and the like of self-emulsifying compositions.

Furthermore, water may be present not only due to active addition but also for passive reasons, such as being derived from the raw materials of self-emulsifying compositions.

The content of the water, relative to the total mass of the self-emulsifying composition, is preferably 30% by mass or less; more preferably 20% by mass or less; and even more preferably 1 to 15% by mass.

Also, the content of water, relative to the total mass of the core particles, is preferably 30% by mass or less; more preferably 20% by mass or less; and even more preferably 1 to 15% by mass. Furthermore, when two or more types of water are contained, it is preferable for their total content to fall within the above ranges.

### (Additive)

The core particle can further comprise additives.

Examples of additives include, but are not limited to, absorption promoters, pH adjusters, coloring agents, masking agents, sweeteners, antioxidants, preservatives, smoothening agents, and fragrances. These additives can be used alone or in combination with two or more types.

### [Coating particle layer]

The coating particle layer of self- emulsifying composition according to the present invention coats the core particles. The coating particle layer comprises coating particles. The coating particles attach onto the surface of the core particles, forming a layered structure which becomes the coating particle layer. The coating particle layer of the present invention is layered, and the layered structure includes the structure in which the coating particle layer completely coats the core particles and the structure in which the coating particle layer partially coats the core particles. In the structure in which the coating particle layer partially coats the core particles, uncoated parts of the core particles are exposed.

The coating particles are not particularly limited as far as they are the particles which can coat the core particles and form a layer, but they are typically water-insoluble particles. Examples of coating particles include divalent or higher metal salts, inorganic substances, polysaccharides or derivatives thereof, and organic polymers.

Examples of the divalent or higher metal salts include calcium chloride, calcium carbonate, calcium oxide, calcium sulfate, calcium hydrogen phosphate, calcium dihydrogen phosphate, calcium stearate, magnesium chloride, magnesium carbonate, magnesium oxide, magnesium silicate, aluminum chloride, aluminum hydroxide, aluminum phosphate, aluminum sulfate, aluminum silicate, potassium aluminum sulfate, iron(III) chloride, ammonium alum, ferrous sulfate, iron citrate, zinc oxide, zinc sulfate, and hydrates thereof.

Examples of the inorganic substances include anhydrous silica, silicon dioxide, titanium oxide, kaolin, diatomaceous earth, bentonite, zeolite, and talc.

Examples of the polysaccharides or derivatives thereof include corn starch, rice starch, wheat starch, potato starch, agar powder, crystalline cellulose, ethyl cellulose, croscarmellose sodium, low-substituted sodium carboxymethyl starch and sodium starch glycolate.

Examples of the organic polymers include crosspovidone, aminoalkyl methacrylate copolymer E, methacrylic acid copolymer L, dried methacrylic acid copolymer LD, methacrylic acid copolymer S, and ammonioalkyl methacrylate copolymer.

Among these, the coating particles of the coating particle layer preferably contain at least one selected from the group consisting of bentonite, ethyl cellulose, corn starch, rice starch, wheat starch, potato starch, calcium stearate, magnesium carbonate, low substituted sodium carboxymethyl starch, sodium starch glycolate, anhydrous silica, magnesium silicate, diatomaceous earth, zeolite, silicon dioxide, agar powder, crosscarmellose sodium, crospovidone, and talc; more preferably contain bentonite and/or corn starch; and even more preferably contain corn starch. Furthermore, the coating particles described above can be used alone or in combination with two or more types.

The mean particle diameter of the coating particle is preferably 1 to 100 µm; more preferably 2 to 50 µm; and even more preferably 3 to 30 µm. When the mean particle diameter of the coating particle is 1 µm or more, it allows for a higher fluidity of coating particles, which is preferable. On the other hand, when the mean particle diameter of the coating particle is 100 µm or less, the reduced surface area of the coating particles can enhance the bonding strength with the core particles, which is preferable.

The content of the coating particle layer, relative to the total mass of the self-emulsifying composition, is preferably 90% by mass or less; more preferably 10 to 85% by mass; even more preferably 15 to 70% by mass, and particularly preferably 20 to 70% by mass. When the content of the coating particle layer is 90% by mass or less, the form stability of the self-emulsifying composition can increase, which is preferable.

### 2 Method of producing self-emulsifying composition

One embodiment of the present invention provides a method of producing self-emulsifying composition. The production method according to the present invention comprises a granulation step of introducing droplets of a granulation solution containing an oily ingredient, a surfactant, and a thickener into a fluidized bed where coating particles flow to attach the coating particles onto the surface of the droplets for granulation. The granulation can be performed by attaching the coating particles onto the surface of droplets of granulation solution, such that the ingredients in the granulation solution (preferably at least one of surfactant, solvent, and water; and more preferably solvent and/or water) is absorbed by the coating particles.

### [Granulation step]

The granulation step can be performed by using the granulation method, for example, disclosed in Japanese Patent Application Laid-Open No. 2019-073459, and specifically the method comprising a step of introducing droplets of a granulation solution containing oily ingredients, surfactants, and thickeners into a fluidized bed where coating particles flow to attach the coating particles onto the surface of the droplets for granulation. More specifically, droplets of the granulation solution can be introduced into a fluidized bed composed of coating particles through intermittent ejection of a predetermined amount of the granulation solution, to attach the coating particles onto the surface of the droplets, such that the ingredients in the granulation solution is absorbed by the coating particles.

### (Granulation solution)

The granulation solution comprises oily ingredients, surfactants, and thickeners. The granulation solution can further comprise useful substances, solvents, water, additives, etc. The oily ingredients, surfactants, and thickeners, etc. comprised in the granulation solution form core particles of the self-emulsifying composition.

The ingredients of oily ingredients, surfactants, thickeners, useful substances, solvents, water, and additives, etc. are as described above.

The contents of each ingredient such as oily ingredients, surfactants, thickeners, useful substances, solvents, water, and additives are preferably adjusted so that their contents in the core particles in the self-emulsifying composition fall within the ranges as described above. Furthermore, by adjusting the content of solids in the granulation solution (the total content of ingredients other than the solvent and water in the granulation solution), it is possible to adjust the content of core particles and the mean particle diameter of the core particles in the self-emulsifying composition.

Specifically, the content of the oily ingredients in the granulation solution, relative to the total mass of the granulation solution, is preferably 1 to 50% by mass; more preferably 1.5 to 40% by mass; and even more preferably 2 to 30% by mass. Furthermore, when two or more types of oily ingredients are contained, it is preferable for their total content to fall within the above ranges.

The content of the surfactants in the granulation solution, relative to the total mass of the granulation solution, is preferably 1 to 60% by mass; more preferably 2 to 50% by mass; and even more preferably 3 to 40% by mass. Furthermore, when two or more types of surfactants are contained, it is preferable for their total content to fall within the above ranges.

The content of the thickeners in the granulation solution, relative to the total mass of the granulation solution, is preferably 30 to 95% by mass; more preferably 30 to 85% by mass; even more preferably 30 to 75% by mass; and particularly preferably 45 to 75% by mass. Furthermore, when two or more types of thickeners are contained, it is preferable for their total content to fall within the above ranges.

Although depending on the type of useful substances, the content of useful substances in the granulation solution, relative to the total mass of the granulation solution, is preferably 0.01 to 3% by mass; more preferably 0.02 to 2% by mass; and even more preferably 0.02 to 1% by mass. Furthermore, when two or more types of useful substances are contained, it is preferable for their total content to fall within the above ranges.

In another embodiment, the content of useful substances in the granulation solution, relative to the total mass of the granulation solution, is preferably 3 to 50% by mass; more preferably 5 to 40% by mass; and even more preferably 10 to 30% by mass.

The content of the solvents in the granulation solution, relative to the total mass of the granulation solution, is preferably 0.5 to 30% by mass; more preferably 1 to 20% by mass; and even more preferably 1 to 15% by mass, 2 to 15% by mass, 5 to 15% by mass, 5 to 9% by mass, 5 to 10% by mass, 9 to 15% by mass, and 10 to 15% by mass. Furthermore, when two or more types of solvents are contained, it is preferable for their total content to fall within the above ranges.

The content of the water in the granulation solution, relative to the total mass of the granulation solution, is preferably 30% by mass or less; more preferably 20% by mass or less; and even more preferably 10% by mass or less.

However, depending on the oily ingredients, the surfactants, the thickeners, the useful substances, the additives, etc., which are used, the content of the water can be increased. For example, when the gelatin is used as thickener, it is preferable that the content of the water in the granulation solution is increased. In one embodiment, the content of the water in the granulation solution, relative to the total mass of the granulation solution, is preferably 40 to 80% by mass; more preferably 50 to 80% by mass; and even more preferably 60 to 80% by mass.

The content of the additives in the granulation solution, relative to the total mass of the granulation solution, is preferably 2 to 40% by mass; more preferably 5 to 30% by mass; and even more preferably 10 to 20% by mass. Furthermore, when two or more types of additives are contained, it is preferable for their total content to fall within the above ranges.

The viscosity of the granulation solution is not particularly limited, but is preferably between 0.5 and 20000 mPa; and more preferably between 1 and 10000 mPa. When the viscosity of the granulation solution is 5 mPa or more, it makes it easier for the droplets of the ejected granulation solution to maintain their form when they are introduced into a fluidized bed, which is preferable. When the viscosity of the granulation solution is 20,000 mPa or less, it improves productivity, which is preferable.

### (Ejection of granulation solution)

The granulation solution is preferably ejected intermittently in predetermined amounts. At the time, the ejected granulation solution takes the form of droplets. Ejection of the granulation solution can be carried out, for example, by a liquid discharge device equipped with a discharge nozzle.

The ejection amount of the granulation solution per one time can be typically controlled by the nozzle diameter and discharge pressure of the discharge nozzle of the liquid discharge device. Furthermore, by adjusting the ejection amount of the granulation solution, the mean particle diameter of the core particles can be controlled.

The nozzle diameter of the discharge nozzle is not particularly limited, but is preferably 0.1 to 1.0 mm; and more preferably 0.2 to 0.5 mm. When the nozzle diameter of the discharge nozzle is 0.1 mm or more, it is preferable from such perspectives that a highly viscous granulating solution can be discharged and the productivity improves. On the other hand, when the nozzle diameter of the discharge nozzle is 1.0 mm or less, the droplet form can be maintained when in contact with the fluidized bed, which is preferable.

The discharge pressure is not particularly limited, but is preferably 0.1 to 5.0 MPa; and more preferably 0.1 to 2.0 MPa. When the discharge pressure is 0.1 MPa or more, it is preferable from such perspectives that a highly viscous granulating solution can be discharged and the productivity improves. On the other hand, when the discharge pressure is 5.0 MPa or less, the droplet form can be maintained when in contact with the fluidized bed, which is preferable.

The ejection interval time (intermittent time) of the granulation solution is typically controlled by the intermittent ejection frequency of the liquid discharge device. The intermittent ejection frequency is not particularly limited, but is preferably 10 to 1000 Hz; and more preferably 20 to 350 Hz. When the intermittent ejection frequency is 10 Hz or more, it improves productivity, which is preferable. On the other hand, when the intermittent ejection frequency is 1000 Hz or less, it can suppress the heat generation of the device due to friction, which is preferable. Furthermore, as used herein, "intermittent ejection frequency" refers to the number of ejections per second.

### (Introduction into fluidized bed)

The droplets of the ejected granulation solution are introduced into a fluidized bed. In the fluidized bed, the coating particles are stored, and when droplets contact with the coating particles in the fluidized bed, the coating particles attach onto the surface of the droplets. Next, the attached coating particles absorb the ingredients from the droplets, resulting in a decrease in the fluidity of the droplets, which then form the core particles. In addition, the coating particles attached to the core particles form a layered structure, becoming a coating particle layer, which enables the production of self-emulsifying compositions. Furthermore, the coating particles are as described above.

Examples of fluidized bed include that is formed by coating particles stored and rolled in a pan granulator. The rotating pan (plate) applies a rolling action to the coating particles, allowing them to form a layered structure when the droplets contact with the coating particles, and preventing or suppressing the collisions with subsequent droplets.

The vibration applied to the coating particles in a fluidized bed is not particularly limited, but is preferably 10 to 500 rpm; and more preferably 20 to 200 rpm. When the vibrations is 10 rpm or higher, it can prevent or suppress collisions with subsequent droplets, which is preferable. On the other hand, when the vibration is 500 rpm or lower, it can suppress the deflection of the coating particles due to centrifugal force, which is preferable.

### 3 solid preparation

One embodiment of the present invention provides solid preparations containing self-emulsifying composition. Examples of dosage forms of the solid preparations are not specifically limited but include tablet, capsule, powder, fine granule, granule. Among these, the solid preparations are preferably tablet or granule; and more preferably tablet.

Tablets are usually formed by compressing self-emulsifying compositions. At the time, along with self-emulsifying compositions, excipients, disintegrants, binders, lubricants, stabilizers, suspending agents, coating agents, antioxidants, dispersing agents, fluidizers, flavors, sweeteners, taste enhancers, colorants, etc. can be added and then compressed.

Furthermore, the solid preparations are preferably oral formulations.

### Examples

Hereinafter, the present invention is specifically explained with reference to Examples, but the present invention is not limited to the following Examples.

### [Self-emulsifying formulation 1]

A self-emulsifying formulation comprising an oily ingredient, a surfactant and a solvent was prepared.

Specifically, a self-emulsifying formulation was prepared by mixing at 70°C; 20 parts by mass of medium-chain fatty acid triglyceride (MCT) as oily ingredient; 24 parts by mass of polyoxyethylene castor oil (POE castor oil) and 36 parts by mass of polyoxyethylene hydrogenated castor oil (POE hydrogenated castor oil) as surfactants; and 20 parts by mass of polyethylene glycol 400 (PEG 400) as solvent.

### [Self-emulsifying formulation 2]

A self-emulsifying formulation comprising an oily ingredient, a surfactant and a solvent was prepared.

Specifically, a self-emulsifying formulation was prepared by mixing at 60°C; 60.6 parts by mass of propylene glycol monocaprate (PG monocaprate) as oily ingredient; 20.2 parts by mass of POE castor oil and 6.4 parts by mass of POE hydrogenated castor oil as surfactants; and 12.8 parts by mass of polypropylene glycol (PG) as solvent.

### [Self-emulsifying formulation 3]

A self-emulsifying formulation comprising an oily ingredient and a surfactant was prepared.

Specifically, a self-emulsifying formulation was prepared by mixing at 60°C; 31.6 parts by mass of propylene glycol monolaurate (PG monolaurate) as oily ingredient; and 52.6 parts by mass of POE castor oil and 15.8 parts by mass of oleyl macrogol-6 glyceride (PEG-6 oleate) as surfactants.

### [Self-emulsifying formulation 4]

A self-emulsifying formulation comprising an oily ingredient, a surfactant and a solvent was prepared.

Specifically, a self-emulsifying formulation was prepared by mixing at 60°C; 15 parts by mass of ethyl oleate as oily ingredient; 21.3 parts by mass of POE castor oil and 42.4 parts by mass of capryloyl macrogol glyceride (PEG caprylate) as surfactants; and 21.3 parts by mass of PG as solvent.

Self-emulsifying formulations 1 to 4 are shown in Table 1 below.

**[Table 1]**

| | oily ingredient | | surfactant | | | | solvent | | total |
|---|---|---|---|---|---|---|---|---|---|
| | type | content (% by mass) | type | content (% by mass) | type | content (% by mass) | type | content (% by mass) | |
| Self-emulsifying formulation 1 | MCT | 20 | POE castor oil | 24 | POE hydrogenated castor oil | 36 | PEG400 | 20 | 100 |
| Self-emulsifying formulation 2 | PG monocaprate | 60.6 | POE castor oil | 20.2 | POE hydrogenated castor oil | 6.4 | PG | 12.8 | 100 |
| Self-emulsifying formulation 3 | PG monolaurate | 31.6 | POE castor oil | 52.6 | PEG-6 oleate | 15.8 | - | 0 | 100 |
| Self-emulsifying formulation 4 | ethyl oleate | 15 | POE castor oil | 21.3 | PEG caprylate | 42.4 | PG | 21.3 | 100 |

### [Granulation solutions 1 to 5]

Granulation solutions comprising an oily ingredient, a surfactant, a thickener and a solvent were prepared.

Granulation solutions were prepared by mixing self-emulsifying formulation 1 and polyethylene glycol 4000 (PEG 4000) as thickener at predetermined content ratios at 70°C.

### [Granulation solution 6]

Polyethylene glycol 4000 (PEG 4000) was used as granulation solution.

### [Granulation solution 7]

Self-emulsifying formulation 1 was used as granulation solution.

### [Granulation solutions 8 to 11]

Granulation solutions comprising an oily ingredient, a surfactant, a thickener and a solvent were prepared.

Granulation solutions were prepared by mixing self-emulsifying formulation 2 and 40% gelatin aqueous solution as thickener at predetermined content ratios at 60°C.

### [Granulation solutions 12]

40% gelatin aqueous solution was used as granulation solution.

### [Granulation solutions 13 to 18]

Granulation solutions comprising an oily ingredient, a surfactant, a thickener and a solvent were prepared.

Granulation solutions were prepared by mixing self-emulsifying formulation 2 and polyoxyl 40 stearate (PEG stearate) as thickener at predetermined content ratios at 80°C.

### [Granulation solution 19]

Polyoxyl 40 stearate (PEG stearate) was used as granulation solution.

### [Granulation solutions 20 to 23]

Granulation solutions comprising an oily ingredient, a surfactant, a thickener and a solvent were prepared.

Granulation solutions were prepared by mixing self-emulsifying formulation 2 and polyoxyethylene polyoxypropylene glycol (POE-POPG) as thickener at predetermined content ratios at 80°C.

### [Granulation solutions 24]

Polyoxyethylene polyoxypropylene glycols (POE-POPG) was used as granulation solution.

### [Granulation solution 25]

Self-emulsifying formulation 2 was used as granulation solution.

### [Granulation solutions 26 to 30]

Granulation solutions comprising an oily ingredient, a surfactant, and a thickener were prepared.

Granulation solutions were prepared by mixing self-emulsifying formulation 3 and polyoxyethylene polyoxypropylene glycol (POE-POPG) as thickener at predetermined content ratios at 80°C.

### [Granulation solution 31]

Self-emulsifying formulation 3 was used as granulation solution.

### [Granulation solutions 32 to 36]

Granulation solutions comprising an oily ingredient, a surfactant, a thickener and a solvent were prepared.

Granulation solutions were prepared by mixing self-emulsifying formulation 4 and polyoxyl 40 stearate (PEG stearate) as thickener at predetermined content ratios at 80°C.

### [Granulation solution 37]

Self-emulsifying formulation 4 was used as granulation solution.

### [Granulation solution 38]

Granulation solution comprising an oily ingredient, a surfactant, a thickener, a useful substance, and a solvent was prepared.

Granulation solution was prepared by mixing at 70°C; 50 parts by mass of self-emulsifying formulation 1; 33 parts by mass of polyethylene glycol 4000 (PEG 4000) as thickener; and 10 parts by mass of tacrolimus (having a logarithmic value of octanol/water partition coefficient (Log P value) of 3 or higher) as useful substance.

Furthermore, it was visually confirmed that a portion of tacrolimus was dissolved in the granulation solution obtained.

### [Granulation solution 39]

Granulation solution comprising an oily ingredient, a surfactant, a thickener, a useful substance, and a solvent was prepared.

Granulation solution was prepared by mixing at 60°C; 50 parts by mass of self-emulsifying formulation 2; 41 parts by mass of 40% gelatin aqueous solution as thickener; and 10 parts by mass of tacrolimus as useful substance.

Furthermore, it was visually confirmed that a portion of tacrolimus was dissolved in the granulation solution obtained.

### [Granulation solution 40]

Granulation solution comprising an oily ingredient, a surfactant, a thickener, a useful substance, and a solvent was prepared.

Granulation solution was prepared by mixing at 80°C; 50 parts by mass of self-emulsifying formulation 2; 117 parts by mass of polyoxyl 40 stearate (PEG stearate) as thickener; and 10 parts by mass of tacrolimus as useful substance.

Furthermore, it was visually confirmed that a portion of tacrolimus was dissolved in the granulation solution obtained.

### [Granulation solution 41]

Granulation solution comprising an oily ingredient, a surfactant, a thickener, a useful substance, and a solvent was prepared.

Granulation solution was prepared by mixing at 80°C; 50 parts by mass of self-emulsifying formulation 2; 75 parts by mass of polyoxyethylene polyoxypropylene glycol (POE-POPG) as thickener; and 10 parts by mass of tacrolimus as useful substance.

Furthermore, it was visually confirmed that a portion of tacrolimus was dissolved in the granulation solution obtained.

### [Granulation solution 42]

Granulation solution comprising an oily ingredient, a surfactant, a thickener, and a useful substance was prepared.

Granulation solution was prepared by mixing at 80°C; 50 parts by mass of self-emulsifying formulation 3; 117 parts by mass of polyoxyethylene polyoxypropylene glycol (POE-POPG) as thickener; and 10 parts by mass of tacrolimus as useful substance.

Furthermore, it was visually confirmed that a portion of tacrolimus was dissolved in the granulation solution obtained.

### [Granulation solution 43]

Granulation solution comprising an oily ingredient, a surfactant, a thickener, a useful substance, and a solvent was prepared.

Granulation solution was prepared by mixing at 80°C; 50 parts by mass of self-emulsifying formulation 4; 50 parts by mass of polyoxyl 40 stearate (PEG stearate) as thickener; and 10 parts by mass of tacrolimus as useful substance.

Furthermore, it was visually confirmed that a portion of tacrolimus was dissolved in the granulation solution obtained.

### [Granulation solution 44]

Granulation solution comprising an oily ingredient, a surfactant, a useful substance, and a solvent was prepared.

Granulation solution was prepared by mixing 50 parts by mass of self-emulsifying formulation 1 and 10 parts by mass of tacrolimus as useful substance at 60°C.

Furthermore, it was visually confirmed that a portion of tacrolimus was dissolved in the granulation solution obtained.

### [Granulation solutions 45]

Granulation solution comprising an oily ingredient, a surfactant, a useful substance, and a solvent was prepared.

Granulation solution was prepared by mixing 50 parts by mass of self-emulsifying formulation 2 and 10 parts by mass of tacrolimus as useful substance at 60°C.

Furthermore, it was visually confirmed that a portion of tacrolimus was dissolved in the granulation solution obtained.

### [Granulation solution 46]

Granulation solution comprising an oily ingredient, a surfactant, and a useful substance was prepared.

Granulation solution was prepared by mixing 50 parts by mass of self-emulsifying formulation 3 and 10 parts by mass of tacrolimus as useful substance at 60°C.

Furthermore, it was visually confirmed that a portion of tacrolimus was dissolved in the granulation solution obtained.

### [Granulation solution 47]

Granulation solution comprising an oily ingredient, a surfactant, a useful substance, and a solvent was prepared.

Granulation solution was prepared by mixing 50 parts by mass of self-emulsifying formulation 4 and 10 parts by mass of tacrolimus as useful substance at 60°C.

Furthermore, it was visually confirmed that a portion of tacrolimus was dissolved in the granulation solution obtained.

Granulation solutions 1 to 47 are shown in Tables 2 to 4 below.

**[Table 2]**

| | self-emulsifying formulation | | thickener | |
|---|---|---|---|---|
| | type | content (% by mass) | type | content (% by mass) |
| Granulation solution 1 | Self-emulsifying formulation 1 | 60 | PEG4000 | 40 |
| Granulation solution 2 | Self-emulsifying formulation 1 | 40 | PEG4000 | 60 |
| Granulation solution 3 | Self-emulsifying formulation 1 | 30 | PEG4000 | 70 |
| Granulation solution 4 | Self-emulsifying formulation 1 | 20 | PEG4000 | 80 |
| Granulation solution 5 | Self-emulsifying formulation 1 | 10 | PEG4000 | 90 |
| Granulation solution 6 | - | 0 | PEG4000 | 100 |
| Granulation solution 7 | Self-emulsifying formulation 1 | 100 | - | 0 |
| Granulation solution 8 | Self-emulsifying formulation 2 | 50 | 40% gelatin aqueous solution | 50 |
| Granulation solution 9 | Self-emulsifying formulation 2 | 40 | 40% gelatin aqueous solution | 60 |
| Granulation solution 10 | Self-emulsifying formulation 2 | 33 | 40% gelatin aqueous solution | 67 |
| Granulation solution 11 | Self-emulsifying formulation 2 | 29 | 40% gelatin aqueous solution | 71 |
| Granulation solution 12 | - | 0 | 40% gelatin aqueous solution | 100 |
| Granulation solution 13 | Self-emulsifying formulation 2 | 50 | PEG stearate | 50 |
| Granulation solution 14 | Self-emulsifying formulation 2 | 40 | PEG stearate | 60 |
| Granulation solution 15 | Self-emulsifying formulation 2 | 33 | PEG stearate | 67 |
| Granulation solution 16 | Self-emulsifying formulation 2 | 29 | PEG stearate | 71 |
| Granulation solution 17 | Self-emulsifying formulation 2 | 20 | PEG stearate | 80 |
| Granulation solution 18 | Self-emulsifying formulation 2 | 10 | PEG stearate | 90 |
| Granulation solution 19 | - | 0 | PEG stearate | 100 |
| Granulation solution 20 | Self-emulsifying formulation 2 | 40 | POE-POPG | 60 |
| Granulation solution 21 | Self-emulsifying formulation 2 | 30 | POE-POPG | 70 |
| Granulation solution 22 | Self-emulsifying formulation 2 | 20 | POE-POPG | 80 |
| Granulation solution 23 | Self-emulsifying formulation 2 | 10 | POE-POPG | 90 |
| Granulation solution 24 | - | 0 | POE-POPG | 100 |
| Granulation solution 25 | Self-emulsifying formulation 2 | 100 | - | 0 |

**[Table 3]**

| | self-emulsifying formulation | | thickener | |
|---|---|---|---|---|
| | type | content (% by mass) | type | content (% by mass) |
| Granulation solution 26 | Self-emulsifying formulation 3 | 50 | POE-POPG | 50 |
| Granulation solution 27 | Self-emulsifying formulation 3 | 40 | POE-POPG | 60 |
| Granulation solution 28 | Self-emulsifying formulation 3 | 30 | POE-POPG | 70 |
| Granulation solution 29 | Self-emulsifying formulation 3 | 20 | POE-POPG | 80 |
| Granulation solution 30 | Self-emulsifying formulation 3 | 10 | POE-POPG | 90 |
| Granulation solution 31 | Self-emulsifying formulation 3 | 100 | - | 0 |
| Granulation solution 32 | Self-emulsifying formulation 4 | 50 | PEG stearate | 50 |
| Granulation solution 33 | Self-emulsifying formulation 4 | 40 | PEG stearate | 60 |
| Granulation solution 34 | Self-emulsifying formulation 4 | 30 | PEG stearate | 70 |
| Granulation solution 35 | Self-emulsifying formulation 4 | 20 | PEG stearate | 80 |
| Granulation solution 36 | Self-emulsifying formulation 4 | 10 | PEG stearate | 90 |
| Granulation solution 37 | Self-emulsifying formulation 4 | 100 | - | 0 |

**[Table 4]**

| | self-emulsifying formulation | | thickener | | useful substance (tacrolimus) |
|---|---|---|---|---|---|
| | type | content (% by mass) | type | content (% by mass) | content (% by mass) |
| Granulation solution 38 | Self-emulsifying formulation 1 | 54 | PEG4000 | 35 | 11 |
| Granulation solution 39 | Self-emulsifying formulation 2 | 50 | 40% gelatin aqueous solution | 41 | 10 |
| Granulation solution 40 | Self-emulsifying formulation 2 | 28 | PEG stearate | 66 | 6 |
| Granulation solution 41 | Self-emulsifying formulation 2 | 37 | POE-POPG | 56 | 7 |
| Granulation solution 42 | Self-emulsifying formulation 3 | 28 | POE-POPG | 66 | 6 |
| Granulation solution 43 | Self-emulsifying formulation 4 | 45.5 | PEG stearate | 45.5 | 9 |
| Granulation solution 44 | Self-emulsifying formulation 1 | 83 | - | 0 | 17 |
| Granulation solution 45 | Self-emulsifying formulation 2 | 83 | - | 0 | 17 |
| Granulation solution 46 | Self-emulsifying formulation 3 | 83 | - | 0 | 17 |
| Granulation solution 47 | Self-emulsifying formulation 4 | 83 | - | 0 | 17 |

### [Evaluation of granulation solutions]

The mean particle diameter and polydispersity index of droplets in the o/w emulsions formed in water were evaluated for granulation solutions 1 to 16, granulation solution 19, granulation solutions 24 to 25, granulation solution 31, and granulation solution 37.

### (Preparation of measurement samples)

Water in an amount which results in a concentration of 1% by mass of the self-emulsifying formulation was added to the granulation solution, and the mixture was heated to 60°C and mixed at 2000 rpm using a vortex mixer to prepare the measurement sample.

### (Mean particle diameter)

The mean particle diameter of droplets in the measurement sample was measured at a temperature of 25°C using the dynamic light scattering method with the Zetasizer Nano ZS (manufactured by Malvern Panalytical), in accordance with the definitions of ISO 13321:1996 (Particle size analysis - Photon correlation spectroscopy: MOD) Annex E and ISO 22412:2017 (Dynamic light scattering (DLS)).

### (Polydispersity index)

The polydispersity index of droplets in the measurement sample was measured at a temperature of 25°C using the Zetasizer Nano ZS (manufactured by Malvern Panalytical), in accordance with the definitions of ISO 13321:1996 (Particle size analysis - Photon correlation spectroscopy: MOD) Annex E and ISO 22412:2017 (Dynamic light scattering (DLS)).

The obtained results are shown in Table 5 below.

**[Table 5]**

| | mean particle diameter (nm) | polydispersity index |
|---|---|---|
| Granulation solution 1 | 65 | 0.28 |
| Granulation solution 2 | 77.8 | 0.28 |
| Granulation solution 3 | 77.6 | 0.29 |
| Granulation solution 4 | 161 | 0.36 |
| Granulation solution 5 | 294 | 0.47 |
| Granulation solution 6 | 17.4 | 0.74 |
| Granulation solution 7 | 32.1 | 0.11 |
| Granulation solution 8 | 184.3 | 0.16 |
| Granulation solution 9 | 186.1 | 0.19 |
| Granulation solution 10 | 174 | 0.16 |
| Granulation solution 11 | 215.4 | 0.16 |
| Granulation solution 12 | 139.3 | 1 |
| Granulation solution 13 | 119 | 0.14 |
| Granulation solution 14 | 14.4 | 0.18 |
| Granulation solution 15 | 14.9 | 0.21 |
| Granulation solution 16 | 11.8 | 0.14 |
| Granulation solution 19 | 88.5 | 0.26 |
| Granulation solution 24 | 53.6 | 0.82 |
| Granulation solution 25 | 90.5 | 0.14 |
| Granulation solution 31 | 18.6 | 0.02 |
| Granulation solution 37 | 26.2 | 0.08 |

Granulation solution 7 (self-emulsifying formulation 1), granulation solution 25 (self-emulsifying formulation 2), granulation solution 31 (self-emulsifying formulation 3), and granulation solution 37 (self-emulsifying formulation 4) all formed o/w emulsions in water, with small mean particle diameter and low polydispersity index of droplets. However, granulation solutions 7, 25, 31, and 37 consist only of self-emulsifying formulations containing oily ingredient, surfactant, and optionally solvent, and are expected to be difficult to produce the self-emulsifying composition according to the present invention.

Granulation solutions 1 to 5, granulation solutions 8 to 11, and granulation solutions 13 to 16 all formed o/w emulsions in water, and their mean particle diameters and polydispersity indexes of droplets were acceptable as self-emulsifying formulations. Granulation solutions 1 to 5, granulation solutions 8 to 11, and granulation solutions 13 to 16 are sol solutions in which a thickener has been added to a self-emulsifying formulation, and therefore are expected to be able to produce the self-emulsifying composition according to the present invention.

### [Example 1]

A self-emulsifying composition was produced.

Specifically, a total of 20 g of the granulation solution 1 was charged in a liquid discharge device (discharge nozzle diameter: 0.3 mm), with the discharge pressure set to 0.5 MPa and the intermittent ejection frequency set to 167 Hz.

An open type-pan granulator (pan diameter: 300 mm) was installed 70 cm below the discharge nozzle, and 250 g of corn starch (mean particle diameter: 10 µm) was stored in the open type-pan granulator. By the revolving vibration of the open type-pan granulator at 90 rpm, a fluidized bed was formed where the corn starch rolled and flowed.

A total of 13.8 g of the granulation solution 1 was intermittently ejected from a liquid discharge device to introduce the droplets of granulation solution into a fluidized bed. The corn starch attached onto the surface of the droplets, absorbed the moisture of the droplets, resulting in the production of the self-emulsifying composition having the core particle derived from the granulation solution and the coating particle layer of corn starch. Furthermore, the self-emulsifying composition and corn starch of the open type-pan granulator were separated by a sieve (mesh size: 106 µm).

### [Examples 2 to 23]

Self-emulsifying compositions were produced using the same method as in Example 1 except for using granulation solutions 2 to 5, granulation solution 8, granulation solutions 16 to 18, granulation solutions 20 to 23, and granulation solutions 26 to 30, instead of granulation solution 1.

Self-emulsifying compositions produced in Examples 1 to 23 are shown in Tables 6 to 9 below.

**[Table 6]**

| | granulation solution | core particle | | | | | | | | | | | coating particle |
|---|---|---|---|---|---|---|---|---|---|---|---|---|---|
| | | oily ingredient | | surfactant | | | | solvent | | thickener | | water | |
| | | type | content (% by mass) | type | conten t (% by mass) | type | content (% by mass) | type | content (% by mass) | type | content (% by mass) | content (% by mass) | content (% by mass) |
| Example 1 | granulation solution 1 | MCT | 2.5 | POE castor oil | 3 | POE hydrogenated castor oil | 4.5 | PEG400 | 2.5 | PEG4000 | 8.3 | 0 | 79.2 |
| Example 2 | granulation solution 2 | MCT | 2.2 | POE castor oil | 2.7 | POE hydrogenated castor oil | 4 | PEG400 | 2.2 | PEG4000 | 16.7 | 0 | 72.2 |
| Example 3 | granulation solution 3 | MCT | 1.9 | POE castor oil | 2.3 | POE hydrogenated castor oil | 3.5 | PEG400 | 1.9 | PEG4000 | 22.7 | 0 | 67.7 |
| Example 4 | granulation solution 4 | MCT | 1.6 | POE castor oil | 1.9 | POE hydrogenated castor oil | 2.8 | PEG400 | 1.6 | PEG4000 | 31.3 | 0 | 60.8 |
| Example 5 | granulation solution 5 | MCT | 0.9 | POE castor oil | 1 | POE hydrogenated castor oil | 1.5 | PEG400 | 0.9 | PEG4000 | 38.5 | 0 | 57.2 |
| Example 6 | granulation solution 8 | PG monocaprate | 14.5 | POE castor oil | 4.8 | POE hydrogenated castor oil | 1.5 | PG | 3.1 | gelatin | 9.5 | 10 | 56.6 |

**[Table 7]**

| | granulation solution | core particle | | | | | | | | | | | coating particle |
|---|---|---|---|---|---|---|---|---|---|---|---|---|---|
| | | oily ingredient | | surfactant | | | | solvent | | thickener | | water | |
| | | type | content (% by mass) | type | content (% by mass) | type | content (% by mass) | type | content (% by mass) | type | content (% by mass) | content (% by mass) | content (% by mass) |
| Example 7 | granulation solution 16 | PG monocaprate | 8 | POE castor oil | 2.6 | POE hydrogenated castor oil | 0.8 | PG | 1.7 | PEG stearate | 30.6 | 0 | 56.3 |
| Example 8 | granulation solution 17 | PG monocaprate | 4.4 | POE castor oil | 1.5 | POE hydrogenated castor oil | 0.5 | PG | 0.9 | PEG stearate | 29 | 0 | 63.7 |
| Example 9 | granulation solution 18 | PG monocaprate | 2 | POE castor oil | 0.7 | POE hydrogenated castor oil | 0.2 | PG | 0.4 | PEG stearate | 30 | 0 | 66.7 |
| Example 10 | granulation solution 20 | PG monocaprate | 9.3 | POE castor oil | 3.1 | POE hydrogenated castor oil | 1 | PG | 2 | POE-POPG | 23.1 | 0 | 61.5 |
| Example 11 | granulation solution 21 | PG monocaprate | 8.8 | POE castor oil | 3 | POE hydrogenated castor oil | 0.9 | PG | 1.8 | POE-POPG | 33.9 | 0 | 51.6 |
| Example 12 | granulation solution 22 | PG monocaprate | 6.4 | POE castor oil | 2.1 | POE hydrogenated castor oil | 0.7 | PG | 1.4 | POE-POPG | 42.5 | 0 | 46.9 |
| Example 13 | granulation solution 23 | PG monocaprate | 3.8 | POE castor oil | 1.2 | POE hydrogenated castor oil | 0.4 | PG | 0.8 | POE-POPG | 55.8 | 0 | 38 |

**[Table 8]**

| | granulation solution | core particle | | | | | | | | | | | coating particle |
|---|---|---|---|---|---|---|---|---|---|---|---|---|---|
| | | oily ingredient | | surfactant | | | | solvent | | thickener | | Water | |
| | | type | content (% by mass) | type | content (% by mass) | type | content (% by mass) | type | content (% by mass) | type | content (% by mass) | content (% by mass) | content (% by mass) |
| Example 14 | granulation solution 26 | PG monolaurate | 6.4 | POE castor oil | 10.6 | PEG-6 oleate | 3.2 | - | 0 | POE-POPG | 20.2 | 0 | 59.6 |
| Example 15 | granulation solution 27 | PG monolaurate | 5.3 | POE castor oil | 8.9 | PEG-6 oleate | 2.7 | - | 0 | POE-POPG | 25.4 | 0 | 57.7 |
| Example 16 | granulation solution 28 | PG monolaurate | 4.5 | POE castor oil | 7.5 | PEG-6 oleate | 2.2 | - | 0 | POE-POPG | 33.1 | 0 | 52.7 |
| Example 17 | granulation solution 29 | PG monolaurate | 3.4 | POE castor oil | 5.7 | PEG-6 oleate | 1.7 | - | 0 | POE-POPG | 43.2 | 0 | 46 |
| Example 18 | granulation solution 30 | PG monolaurate | 2.1 | POE castor oil | 3.5 | PEG-6 oleate | 1.1 | - | 0 | POE-POPG | 60.6 | 0 | 32.7 |

**[Table 9]**

| | granulation solution | core particle | | | | | | | | | | | coating particle |
|---|---|---|---|---|---|---|---|---|---|---|---|---|---|
| | | oily ingredient | | surfactant | | | | solvent | | thickener | | water | |
| | | type | content (% by mass) | type | content (% by mass) | type | content (% by mass) | type | content (% by mass) | type | content (% by mass) | content (% by mass) | content (% by mass) |
| Example 19 | granulation solution 32 | oleic acid ethyl | 1.9 | POE castor oil | 2.7 | PEG caprylate | 5.4 | PG | 2.7 | PEG stearate | 12.8 | 0 | 74.5 |
| Example 20 | granulation solution 33 | oleic acid ethyl | 1.9 | POE castor oil | 2.6 | PEG caprylate | 5.3 | PG | 2.6 | PEG stearate | 18.6 | 0 | 69 |
| Example 21 | granulation solution 34 | oleic acid ethyl | 1.5 | POE castor oil | 2.2 | PEG caprylate | 4.4 | PG | 2.2 | PEG stearate | 23.9 | 0 | 65.8 |
| Example 22 | granulation solution 35 | oleic acid ethyl | 1.1 | POE castor oil | 1.5 | PEG caprylate | 3.1 | PG | 1.5 | PEG stearate | 28.9 | 0 | 63.9 |
| Example 23 | granulation solution 36 | oleic acid ethyl | 0.6 | POE castor oil | 0.8 | PEG caprylate | 1.7 | PG | 0.8 | PEG stearate | 35.4 | 0 | 60.7 |

### [Evaluation of self-emulsifying compositions]

The mean particle diameter and polydispersity index of droplets in the o/w emulsions formed in water were evaluated for self-emulsifying compositions produced in Examples 1 to 23.

### (Preparation of measurement samples)

Water in an amount which results in a concentration of 1% by mass of the self-emulsifying formulation was added to the self-emulsifying composition, and the mixture was heated to 60°C and mixed at 2000 rpm using a vortex mixer to prepare the measurement sample.

### (Mean particle diameter)

The mean particle diameter of droplets in the measurement sample was measured at a temperature of 25°C using the dynamic light scattering method with the Zetasizer Nano ZS (manufactured by Malvern Panalytical), in accordance with the definitions of ISO 13321:1996 (Particle size analysis - Photon correlation spectroscopy: MOD) Annex E and ISO 22412:2017 (Dynamic light scattering (DLS)).

### (Polydispersity index)

The polydispersity index of droplets in the measurement sample was measured at a temperature of 25°C using the Zetasizer Nano ZS (manufactured by Malvern Panalytical), in accordance with the definitions of ISO 13321:1996 (Particle size analysis - Photon correlation spectroscopy: MOD) Annex E and ISO 22412:2017 (Dynamic light scattering (DLS)).

The obtained results are shown in Table 10 below.

**[Table 10]**

| | mean particle diameter (nm) | polydispersity index |
|---|---|---|
| Example 1 | 123.3 | 0.54 |
| Example 2 | 199.1 | 0.6 |
| Example 3 | 270.5 | 0.55 |
| Example 4 | 321.6 | 0.55 |
| Example 5 | 512.3 | 0.36 |
| Example 6 | 169.9 | 0.19 |
| Example 7 | 12.1 | 0.09 |
| Example 8 | 11.8 | 0.1 |
| Example 9 | 12.4 | 0.2 |
| Example 10 | 177.4 | 0.15 |
| Example 11 | 231.2 | 0.11 |
| Example 12 | 373.8 | 0.1 |
| Example 13 | 701.4 | 0.1 |
| Example 14 | 266 | 0.46 |
| Example 15 | 206.3 | 0.26 |
| Example 16 | 167 | 0.19 |
| Example 17 | 164 | 0.27 |
| Example 18 | 142.6 | 0.56 |
| Example 19 | 16.1 | 0.13 |
| Example 20 | 14.4 | 0.08 |
| Example 21 | 14.1 | 0.16 |
| Example 22 | 12.4 | 0.14 |
| Example 23 | 13.1 | 0.34 |

Granulation solutions 1 to 5, granulation solution 8, granulation solutions 16 to 18, granulation solutions 20 to 23, granulation solutions 26 to 30, and granulation solutions 32 to 36 are sol solutions containing a thickener, and were able to produce a self-emulsifying composition according to the present invention. Furthermore, granulation solutions 7, 25, 31, and 37, which consist only of self-emulsifying formulation and do not contain thickener, were not able to produce a self-emulsifying composition according to the present invention.

Also, from the results in Table 9, the self-emulsifying compositions of Examples 1 to 23 all formed o/w emulsions in water, and their mean particle diameters and polydispersity indexes of droplets were acceptable as self-emulsifying formulations. Therefore, when the self-emulsifying composition further comprises useful substances in the core particles, these useful substances can, for example, be dissolved or dispersed in oily ingredients and the like, thus stabilizing the absorption behavior and preventing the occurrence of inter-individual variability through oral administration of the self-emulsifying composition.

### [Examples 24 to 29]

Self-emulsifying compositions comprising useful substance (tacrolimus) were produced.

Specifically, self-emulsifying compositions were produced using the same method as in Example 1 except for using granulation solutions 38 to 43, instead of granulation solution 1.

Self-emulsifying compositions comprising useful substance produced in Examples 24 to 29 are shown in Table 11 below.

**[Table 11]**

| | granulation solution | core particle | | | | | | | | | | | | coating particle |
|---|---|---|---|---|---|---|---|---|---|---|---|---|---|---|
| | | oily ingredient | | surfactant | | | | solvent | | thickener | | useful substance | water | |
| | | type | content (% by mass) | type | content (% by mass) | type | content (% by mass) | type | content (% by mass) | type | content (% by mass) | content (% by mass) | content (% by mass) | content (% by mass) |
| Example 24 | granulation solution 38 | MCT | 2.2 | POE castor oil | 2.6 | POE hydrogenated castor oil | 4.0 | PEG 400 | 2.2 | PEG4 000 | 8.1 | 2.4 | 0 | 77.4 |
| Example 25 | granulation solution 39 | PG monocaprate | 13.8 | POE castor oil | 4.6 | POE hydrogenated castor oil | 1.5 | PG | 2.9 | gelatin | 16.7 | 4.1 | 10 | 48.8 |
| Example 26 | granulation solution 40 | PG monocaprate | 7.7 | POE castor oil | 2.6 | POE hydrogenated castor oil | 0.8 | PG | 1.6 | PEG stearate | 29.8 | 2.6 | 0 | 54.9 |
| Example 27 | granulation solution 41 | PG monocaprate | 9.1 | POE castor oil | 3.0 | POE hydrogenated castor oil | 1.0 | PG | 1.9 | POE-POPG | 22.4 | 3.0 | 0 | 59.6 |
| Example 28 | granulation solution 42 | PG monolaurate | 4.4 | POE castor oil | 7.3 | PEG-6 oleate | 2.2 | - | 0 | POE-POPG | 32.2 | 2.8 | 0 | 51.1 |
| Example 29 | granulation solution 43 | oleic acid ethyl | 1.9 | POE castor oil | 2.7 | PEG caprylate | 5.3 | PG | 2.6 | PEG stearate | 12.5 | 2.5 | 0 | 72.5 |

### [Evaluation of self-emulsifying compositions comprising useful substance (tacrolimus)]

The solubility of tacrolimus in water was evaluated when using self-emulsifying compositions comprising useful substance (tacrolimus) produced in Examples 24 to 29.

Furthermore, for comparison, in addition to the self-emulsifying compositions comprising useful substance produced in Examples 24 to 29, granulation solutions 44 to 47 which do not comprise a thickener, as well as a mixed solution of tacrolimus and water (concentration: 0.2% by mass) (reference example) were also evaluated.

### (Preparation of measurement samples)

For the self-emulsifying compositions comprising useful substance from Examples 24 to 29 and granulation solutions 44 to 47, the self-emulsifying formulations were weighed in an equal amount and added with water of 100 times that amount (by mass ratio). The mixtures were then heated to 60°C and mixed at 2000 rpm using a vortex mixer. The obtained mixed solutions were centrifuged at 1500 × g for 5 minutes, after which the supernatants were collected to prepare the measurement samples.

For the reference example, the mixed solution of tacrolimus and water (concentration: 0.2% by mass) was heated to 60°C and mixed at 2000 rpm using a vortex mixer. The obtained mixed solution was centrifuged at 1500 × g for 5 minutes, after which the supernatant was collected to prepare the measurement sample.

### (Measurement of tacrolimus concentration)

The concentrations of tacrolimus in the measurement samples (the concentration of tacrolimus in water) were measured using high-performance liquid chromatography (HPLC). The measurement conditions of HPLC are as follows. The obtained results are shown in Table 12 below.
Column: Octadecylsilyl silica gel (particle diameter 5 µm), inner diameter 4.6 mm, length 15 cm
Column temperature: 50°C
Mobile phase: water / 2-propanol / tetrahydrofuran mixed solution (5:2:2)
Flow rate: 1 mL/min
Detector: Ultraviolet spectrophotometer (measuring wavelength: 220 nm)

**[Table 12]**

| | concentration of tacrolimus (µg/mL) |
|---|---|
| Example 24 | 69.8 |
| Example 25 | 274.2 |
| Example 26 | 125.1 |
| Example 27 | 198.9 |
| Example 28 | 97.7 |
| Example 29 | 88.6 |
| Granulation solution 44 | 82.6 |
| Granulation solution 45 | 811.7 |
| Granulation solution 46 | 104.6 |
| Granulation solution 47 | 75.7 |
| Reference example | 14.2 |

From the results in Table 12, in the granulation solutions 44 to 47, tacrolimus was suitably dissolved in water by the effect of the self-emulsifying formulations. However, granulation solutions 44 to 47 are non-viscous liquid without comprising a thickener, and cannot be made into a self-emulsifying composition, and thus cannot be administered as a solid preparation.

It was found that the self-emulsifying compositions comprising useful substances of Examples 24 to 29 formed o/w emulsions in water, and that the tacrolimus was suitably dissolved in water by the effect of the ingredients contained in the self-emulsifying compositions. Therefore, it is considered that, when a solid preparation of the self-emulsifying composition comprising poorly water-soluble useful substances is administered, the absorption behavior stabilizes, preventing the occurrence of inter-individual variability, as well as suitably absorbing the poorly water-soluble useful substances.

For the reference example, it was confirmed that the solubility of tacrolimus in water at room temperature (25°C) is 2 to 3 µg/mL, and that the solubility remains low even when mixed before concentration measurement.

### Reference Signs List

- 1: core particle
- 2: coating particle layer
- 10: self-emulsifying composition

## Claims

1. A self-emulsifying composition comprising:
a core particle comprising an oily ingredient, a surfactant and a thickener; and
a coating particle layer which coats the core particle.

2. The self-emulsifying composition according to claim 1, wherein the oily ingredient contains at least one selected from the group consisting of hydrogenated vegetable oils, medium-chain fatty acid triglycerides (MCT), long-chain fatty acid alkyl esters, and propylene glycol mono medium-chain fatty acid esters.

3. The self-emulsifying composition according to claim 1, wherein the surfactant contains at least one selected from the group consisting of polyoxyethylene fatty acid glyceryl esters, polyoxyethylene hydrogenated castor oils, and polyoxyethylene castor oils.

4. The self-emulsifying composition according to claim 1, wherein the thickener contains at least one selected from the group consisting of natural thickeners, high molecular weight PEG, glycerin fatty acid esters, polyoxyethylene monoesters, and polyoxyethylene polyoxypropylene glycols (POE-POPG).

5. The self-emulsifying composition according to claim 1, further comprising a useful substance.

6. The self-emulsifying composition according to claim 5, wherein the useful substance has a molecular weight of 500 or more.

7. The self-emulsifying composition according to claim 5, wherein the useful substance has a logarithmic value of octanol/water partition coefficient (Log P value) of 3 or higher.

8. The self-emulsifying composition according to claim 1, further comprising a solvent.

9. The self-emulsifying composition according to claim 8, wherein the solvent contains at least one selected from the group consisting of low molecular weight PEG and polyalcohol.

10. A solid preparation comprising the self-emulsifying composition according to any one of claims 1 to 9.

11. The solid preparation according to claim 10, which is tablet or granule.

12. A method of producing a self-emulsifying composition, comprising a granulation step of introducing droplets of a granulation solution comprising an oily ingredient, a surfactant, and a thickener into a fluidized bed where coating particles flow to attach the coating particles onto the surface of the droplets for granulation.
